**Europäisches Patentamt**

**European Patent Office**

**Office européen des brevets**

(19)

(11) **EP 0 780 479 A2**

(12) **EUROPÄISCHE PATENTANMELDUNG**

(43) Veröffentlichungstag:
25.06.1997 Patentblatt 1997/26

(51) Int. Cl.⁶: **C12Q 1/68**

(21) Anmeldenummer: 96120480.7

(22) Anmeldetag: 19.12.1996

(84) Benannte Vertragsstaaten:
**AT CH DE ES FR GB IT LI NL**

(30) Priorität: **23.12.1995 DE 19548680**

(71) Anmelder: **BOEHRINGER MANNHEIM GMBH**
**68298 Mannheim (DE)**

(72) Erfinder:
• **Leying, Hermann, Dr.**
**83673 Bichl (DE)**
• **Hinzpeter, Matthias, Dr.**
**81375 München (DE)**
• **Wittor, Heiko**
**82327 Tutzing (DE)**
• **Fritton, Hans-Peter, Dr.**
**69509 Mörlenbach (DE)**

(54) **Verfahren zum quantitativen Nachweis von Spezifischen Nukleinsäuresequenzen**

(57) Verfahren bzw. Kit zum quantitativen Nachweis von spezifischen Oligo- bzw. Polynukleotidsequenzen, dadurch gekennzeichnet, daß eine RNA oder einzelsträngige DNA enthaltene Probenmischung mit (einer) zu der zu bestimmenden Nukleotidsequenz komplementären Oligo- bzw. Polynukleotid-Probe(s), die eine spezifisch bindbare und eine detektierbare chemische Gruppe trägt (tragen), hybridisiert wird, anschließend mit einem einzelsträngige Polynukleotidsequenzen spaltenden Agenz vermischt und nach Transfer in ein geeignetes Reaktionsgefäß die immobilisierte oder nicht-immobilisierte Nukleotidprobe bestimmt wird. Als besonders vorteilhalt hat sich erwiesen, wenn eine Mischung bestehend aus unterschiedlichen Spaltagenzien verwendet wird.

EP 0 780 479 A2

**Beschreibung**

Die Erfindung beschreibt ein Verfahren zum Nachweis bzw. zur quantitativen Bestimmung von spezifischen Oligo- bzw. Polynukleotidsequenzen, das im wesentlichen dadurch gekennzeichnet ist, daß eine in einer Mischung, wie z.B. einer biologischen Probe vorliegende einzel- oder doppelsträngige Nukleinsäure, insbesondere mRNA, in Lösung mit einer im wesentlichen zur zu bestimmenden Sequenz komplementären Oligo- bzw. Polynukleotidsequenz hybridisiert, mit einem vorzugsweise einzelsträngige Oligo- bzw. Polynukleotidsequenzen spaltenden Agenz behandelt, anschließend an eine Festphase immobilisiert und die Menge an gebundenem Hybrid bestimmt wird. Als besonders geeignet hat sich erwiesen, wenn das Spaltreagenz eine Mischung zwei unterschielicher RNasen darstellt und die Bindung an die beschichtete Festphase mittels einer spezifisch bindbaren chemischen Gruppe gekoppelt an die komplementäre Nukleotidsequenz(en) und die Detektion mittels einer zweiten chemischen Gruppe erfolgt.

Eine Reihe von Verfahren zum Nachweis von Nukleinsäuren sind heute bekannt. Diese beruhen in der Regel auf dem Prinzip der Hybridisierung, wobei in den meisten Fällen zunächst die Immobilisierung der zu bestimmenden Sequenz an die Festphase erfolgt und anschließend eine markierte Nukleinsäure-Probe zugegeben wird. Das Verfahren ist jedoch zeitaufwendig und für den ungeübten Praktiker nicht ohne weiteres mit Erfolg durchzuführen. Dies gilt insbesondere deshalb, da eine Hybridisierung an der Festphase wenig effizient verläuft.

Alternativ kann die Bestimmung von Nukleinsäuren über die in situ-Markierung der Proben-Nukleinsäure und die Fixierung an die Festphase, vermittelt über eine sequenzspezifische Nukleotidsequenz-Probe, erfolgen. In einem weiteren Verfahren werden zwei sequenzspezifische Probes für die zu bestimmende Nukleinsäure herangezogen. Sowohl die in situ-Markierung, als auch die Hybridisierung mit zwei Probes an der Festphase verlaufen oft nicht reproduzierbar, d.h. sind schwer oder nur mit großer Ungenauigkeit quantifizierbar, sind dazu experimentell aufwendig und somit für die Routine der klinischen Diagnostik wenig geeignet. Entsprechende Verfahren bzw. Varianten sind als Northern-Blot-Verfahren, Nuclease-Protection-Assay und quantitative RT-PCR-Verfahren bekannt und gehören heute zu den Standardmethoden zur Quantifizierung von Nukleinsäuren (T. Maniatis, Molecular Cloning: A Laboratory Manual, 2nd ed. (1989); R. E. Farell, RNA Modologies: A Laboratory Guide for Isolation and Characterization, Academic Press; J. W. Larrick, Trends Biotechnol 10, 146-152 (1992); E. S. Kawasaki, A Guide to Methods and Applications (eds. Innis, M.A. et al) Academic Press). Nachteilig beim Northern-Blot-Verfahren und bei der quantitativen RT-PCR ist, daß zwingend von isolierter RNA ausgegangen werden muß. Bei den Blotting-Verfahren ist insbesondere der Nukleinsäuretransfer und die Immobilisierung der Nukleinsäure unvollständig. Beim klassischen Nuclease-Protection-Assay ist außerdem eine Inaktivierung der Nucleasen durch Protease-Verdau erforderlich.

Außerdem ist der Nachweis von Nukleinsäuren, insbesondere von einzelsträngigen Nukleinsäuren wie z.B. mRNA im sogenannten Mikrotiterplatten-Verfahren bekannt, wobei die Hybridisierungsreaktion in Lösung erfolgt. In der Regel erfolgt dabei die Hybridisierung mit einer Biotin-markierten cDNA-Probe. Die Nukleinsäurehybride werden anschließend über die Biotinmarkierung immobilisiert und beispielsweise mit einem Antikörper, der spezifisch DNA/RNA-Hybride bindet in einem herkömmlichen ELISA-Verfahren detektiert (F. Coutlee et al, J. Biol. Chem. 256, 11601-11604 (1990); EP 0 336 454 u. a.).

Nachteilig bei diesen Verfahren ist jedoch, daß sie zeitaufwendig sind und im ersten Fall lediglich DNA als Fangprobe verwendet werden kann. Dies ist bedingt durch die Tatsache, daß der Nachweis über DNA/RNA-spezifische Antikörper erfolgt. Darüber hinaus sind die Systeme nur wenig sensitiv.

Auch ein erst kürzlich publiziertes Verfahren, bei dem RNA zunächst mit einer bereits im Mikrotiterplatten-well immobilisierten Fangprobe hybridisiert und anschließend mit einem fluoreszierenden interkalierendem Agenz markiert und detektiert wird (T. Okamoto et al, Anal. Biochem. 221, 202-204 (1994)), überkommt die Nachteile nur zum Teil. In Abhängigkeit von der Länge der Fangprobe führt dieses Verfähren zu hohem Hintergrundsignalen, da nicht nur die eigentlich nachzuweisende RNA, sondern auch die immobilisierte Fangprobe markiert wird.

Aufgabe der zugrundeliegenden Erfindung ist daher, ein Verfahren zur Bestimmung einer spezifischen Oligo- bzw. Polynukleotidsequenz zur Verfügung zu stellen, durch das die Nachteile der im Stand der Technik beschriebenen Verfahren überwunden werden, d.h. das insbesondere leicht durchführbar und automatisierbar ist, ohne vorherige RNA-Isolierung (in Zelllysaten) durchführbar ist und mit dem Nukleinsäuren quantitativ erfaßt werden können und das sich darüber hinaus durch geringe Hintergrundssignale auszeichnet.

Gelöst wird die Aufgabe durch ein Verfahren zur Bestimmung einer spezifischen Oligo- bzw. Polynukleotidsequenz in einer Probenmischung, welches folgende Schritte umfaßt:

-   Vereinigen einer RNA oder einzelsträngige DNA enthaltenden Probenmischung mit einem Lysis- bzw. Hybridisierungspuffer,

-   In Kontaktbringen der homogenisierten Probenlösung mit einer oder mehreren, bevorzugt zwei zu der zu bestimmenden Oligo- bzw. Polynukleotidsequenz im wesentlichen komplementären Oligo- bzw. Polynukleotidprobe(s) bzw. entsprechende Derivate, insbesondere Peptidderivate, wobei mindestens zwei unterschiedliche Markierungsreste an dieselbe Probe oder an zwei oder mehrere unterschiedliche Probes gebunden sind,

- Hybridisieren unter stringenten Bedingungen bei einer Temperatur von ungefähr 30° bis 80°C und innerhalb von ca. 10 Minuten bis 15 Stunden,

- Verdünnen der erhaltenen Mischung mit einem geeigneten Puffer und Zusatz eines vorzugsweise einzelsträngigen Oligo- bzw. Polynukleotidsequenzen in Mono- bzw. Oligonukleotide spaltenden Agenz,

- Transfer der Mischung in ein Reaktionsgefäß bzw. in eine Reaktionsmulde, dessen/deren Innenseite mit einer zumindest eine der gebundenen Markierungsreste bindenden Substanz beschichtet ist,

- Immobilisierung des mit zwei unterschiedlichen Markierungsgruppen bzw. einer Markierungsgruppe modifizierten Oligo- bzw. Polynukleotidhybrids an die Festphase und anschließendes Waschen, und

- Detektion der immobilisierten markierten Oligo- bzw. Polynukıeotidprobe oder der nicht-immobilisierten markierten Nukleotidprobe, wobei die Detektion direkt oder beispielsweise über einen geeigneten Enzym-markierten Antikörper erfolgt.

Die komplementäre Oligo- bzw. Polynukleotidsequenz, d.h. Sondensequenz ist mit mindestens zwei unterschiedlichen Markierungsresten verbunden, wovon einer eine spezifisch bindbare chemische Gruppe und der bzw. die weiteren Rest(e) beliebige detektierbare chemische Gruppen darstellen. In diesem Fall wird das im weiteren Verlauf über eine Markierungsgruppe immobilisierte Oligo- bzw. Polynukleinsäure-Hybrid detektiert. Es ist jedoch ach möglich, daß mehrere komplementäre Sondensequenzen in dem erfindungsgemäßen Verfahren eingesetzt werden. In einem solchen Fall trägt eine der Sondensequenzen eine spezifisch bindbare und zumindest eine andere spezifische Sondensequenz eine oder mehrere bestimmbare Gruppen. In dieser analogen Ausführungsform wird das sich in Lösung befindliche spezifische Nukıeinsäurehybrid detektiert. In beide Varianten kann dann auf die Menge der spezifischen Nukleinsäure in einem bestimmten Probenvolumen geschlossen werden. Als immobilisierbare, d.h. spezifisch bindbare chemische Gruppen haben sich insbesondere Biotin, Imminobiotin, Avidin oder andere Streptavidin bindende Moleküle oder Haptene, wie beispielsweise Digoxigenin als vorteilhaft erwiesen. Diese und weitere bindbare Reste sind dem Fachmann bekannt.

Für die Markierung der komplementären Sondensequenz/Sondensequenzen kommen als bestimmbare bzw. detektierbare, enzymatisch aktive Gruppen wie beispielsweise Peroxidase, alkalische Phosphatase oder β-Galactosidase, fluoreszierende Gruppen wie Fluoreszein oder entsprechende Derivate, Chromophore oder lumineszierende Gruppen verschiedenster Alt oder detektierbare Haptene wie beispielsweise Digoxigenin in Betracht. Diese chemischen Gruppen können auf chemischem oder enzymatischem Weg in die Nukleinsäure eingebaut werden. Aber auch Radioisotope, beispielsweise eingebaut in Gegenwart einer terminalen Transferase bzw. T4 RNA-Ligase und eines entsprechend markierten Nukleotids bzw. Oligonukleotids, haben sich als geeignet erwiesen.

Außerdem kann ein Verfahren zum Einführen von nicht-radioaktiv markiertem Desoxynukleotiden in Nukleinsäuren bzw. RNA-Molekülen, die an ihrem 3'-Ende mindestens ein Desoxynukleotid enthalten, das eine nicht-radioaktive Markierungsgruppe trägt, verwendet werden. Ein entsprechendes Verfahren ist in der europäischen Patentanmeldung, Aktenzeichen 95 102 669.9, beschrieben.

Als besonders vorteilhaft hat sich erwiesen, wenn die Markierung des komplementären Oligo- bzw. des Polynukleotids mit einem entsprechendem Hapten, wie beispielsweise Biotin oder Digoxigenin, welches komplexiert in eine Platin-enthaltende Verbindung wie beispielsweise {Pt(ethylendiamin)(Me$_2$SO)(hapten-NH(CS) NHCH$_3$}, durchgeführt wird. Für die Markierung wird eine entsprechend aktivierte Form solcher Platin-Komplexe verwendet. Solche Platin-Verbindungen werden üblicherweise als "Universal Linkage System" (ULS) bezeichnet (EP 0 539 466 / WO 92/01699).

Das Verhältnis von Detektions- zu Bindelabel ist in den meisten Fällen unproblematisch. Es hat sich jedoch als vorteilhaß erwiesen, wenn der Bindelabel im Unterschuß vorliegt; ein Verhältnis von Detektions- zu Bindelabel von 10 zu 1 ist besonders vorteilhaft.

Eine weitere bevorzugte Ausführungsform der Erfindung ist, wenn anstatt einer markierten, komplementären Oligo- bzw. Polynukleotid-Sonde ein Peptid-Nukleinsäure-Derivat mit im wesentlichen zur bestimmenden Sequenz komplementären Basensequenz verwendet wird.

Das heißt zur Hybridisierung können alle Arten von Probes beliebiger Länge verwendet werden, insbesondere anti-sense RNA und sogenannte "Peptide Nucleic Acid" (PNA), d.h. Nukleinsäuren mit Peptid-Backbone. Dies ist von Bedeutung, da die Hybridisierung zwischen PNA- und RNA-Molekülen effizienter erfolgt als zwischen reinem RNA-Molekülen und diese wiederum effizienter hybridisieren als DNA- und RNA-Moleküle. Die Länge der spezifischen Probes ist unproblematisch. In der Regel werden Probes mit Längen von ca. 10 bis 8000 Nukleotiden verwendet, bevorzugt handelt es sich um 100 bis 1000 mere, oft sind Oligo- bzw. Polynukıeotide bestehend aus ca. 100 Nukleotiden ausreichend.

Als zu analysierendes Probematerial kommen neben reinen Nukıeinsäure-Fraktionen, wie insbesondere mRNA-Fraktionen, natürliche oder artifizielle Mischungen von Gesamt-RNA (rRNA, tRNA, mRNA), aber auch aus Zellkulturen gewonnene Fraktionen (Zelllysate) sowie Gewebezellextrakte bzw. Gewebehomogenate sowie Blut menschlichen oder tierischen Ursprungs sowie pflanzliche Extrakte in Betracht.

Geeignete Lysis- bzw. Hybridisierungspuffer basieren auf Puffersubstanzen, die eine gute Pufferkapazität zwischen pH-Wert-Bereich von ca. 2 bis 10, vorzugsweise zwischen pH 7,0 und 8,5 aufweisen. Entsprechende Puffersubstanzen sind beispielsweise Tris • HCl, HEPES, MOPS oder Tris • Borat.

Des weiteren können für das erfindungsgemäße Verfahren geeignete Puffer ein Disulfid reduzierendes Reagenz, wie z.B. Dithioerythritol, Dithiothreitol, Mercaptoethanol, vorzugsweise in einer Konzentration von 0,01 bis 1% (w/V) enthalten. Außerdem hat sich als vorteilhalt erwiesen, wenn das Puffersystem eine denaturierende Substanz, wie Deterengenzien in relativ hohen Konzentrationen enthält. Besonders geeignet haben sich hier Dodecylsulfatsalze oder entsprechende Abkömmlinge in einer Konzentration von 0,1 bis 15% (w/v) sowie, insbesondere bei Einsatz von RNase-reichem Gewebe, Salze vom Guanidiniumthiocyanat (GTC)-Typ bzw. entsprechende Derivate und zwar in einem Konzentrationsbereich von ca. 1 bis 7M. Es hat sich dabei gezeigt, daß es von besonderem Vorteil ist, wenn die Lösungen für die Bestimmungen annähernd frei von RNase-Aktivität sind. Dies bedeutet, daß maximal 5% Restaktivität an RNase vorhanden sein dürfen.

Die für das erfindungsgemäße Verfahren einsetzbaren Puffersysteme können darüber hinaus weitere Salze, wie z.B. Lithiumchlorid oder sonstige Hilfsstoffe enthalten. Als vorteilhaft hat sich zudem erwiesen, wenn zusätzlich ein RNase-Inhibitor, wie z.B. ein aus Placenta gewonnener, zugegen ist und/oder die Pufferlösung zuvor sterilisiert bzw. mit Dimethyldicarbonat oder Diethylpyrocarbonat dekontaminiert wurde.

Die Hybridisierung erfolgt - nach gegebenenfalls stattgefundener Denaturierung - unter stringenten Bedingungen (s. z.B. Analyt. Biochem 163, 281-291 (1987)). Insbesondere haben sich hier Temperaturen zwischen 30° und 80°C und der Zusatz von GTC, bevorzugt in einer Konzentration von 4 M, als geeignet erwiesen. Die Zeit ist in weiten Grenzen variierbar (ca. 10 Minuten bis 24 Stunden), d.h. problemlos. Als ideal hat sich in vielen Fällen eine Inkubationszeit von 4 bis 12 Stunden erwiesen.

Nach der Hybridisierung wird die Probenmischung, in der sich das spezifische Hybrid sowie einzelsträngige, nicht hybridisierte Nukleinsäuren befinden, mit einem geeigneten Puffer, wie z.B. sogenannten RNaseverdaupuffer (z.B. Tris • HCl/EDTA/Natriumacetat, pH 7,5) verdünnt. Verdünnungen von 1:2 bis 1:100, insbesondere ein Verhältnis von 1:10 bis 1:20 haben sich hier als geeignet erwiesen.

Anschließend wird ein Agenz, welches vorzugsweise einzelsträngige, d.h. nicht hybridisierte Nukleinsäuresequenzen in Mono- bzw. Oligonukleotide spaltet, in einer Endkonzentration von ca. 0,01 U/ml bis 50000 U/ml, bevorzugt von ca. 0,05 bis 20000 U/ml zu der Mischung gegeben. Als RNasen kommen insbesondere RNase A, RNase T1 oder RNase I oder entsprechende Mischungen dieser Enzyme in Frage. Als besonders bevorzugt haben sich hier Mischungen bestehend aus unterschiedlichen Spaltagenzien erwiesen. Insbesondere solche Mischungen, in denen von einem Typ Spaltagenz lediglich ein Bruchteil der Menge des/der anderen Spaltagenzien vorliegen, haben sich als ganz besonders geeignet erwiesen. Der Bruchteil kann zwischen 1/5 bis maximal 1/100000 der Gesamtmenge an Spaltagenz ausmachen. Vorteilhalte Konzentrationsbereiche sind für die unterschiedlichen Spaltagenzien beispielsweise: 0,01 bis 10000 U/ml RNase A, 0,05 bis 50000 U/ml RNase T1 oder 0,1 bis 10 U/ml RNase I, jeweils bezogen auf das Endvolumen.

In der Regel sind für die Verdaureaktion wenige Minuten ausreichend; eine Inkubationszeit von mehreren Stunden hat sich jedoch nicht als schädlich erwiesen. Besonders bevorzugt hat sich hier ein Inkubationszeit von ca. 2 Minuten bis ca. 3 Stunden und ein Temperaturbereich von 20° bis 50°C erwiesen. Eine Temperatur von ca. 37°C war in vielen Fällen ideal und in der Regel lagen die erforderlichen Temperaturen nicht unter ca. 10°C und nicht über ca. 80°C.

Die Mischung wird anschließend in ein Separates Reaktionsgefäß überführt. Das Reaktionsgefäß besteht aus einer beschichteten Festphase, wobei die Festphase prinzipiell aus einer Reihe von Materialien und Formen bestehen, wie z.B. Mikropartikel, sogenannte Beads, porenhaltige oder nichtpermeable Membranen, der inneren Oberflächen von Reaktionsgefäßen wie Teströhrchen oder Mikrotiterplatten. Bevorzugt wird die vorliegende Erfindung an beschichteten Mikrotiterplatten durchgeführt, insbesondere solche, bei denen die Beschichtung mit Streptavidin (SA) oder Avidin vorgenommen wurden. Entsprechende Maßnahmen bzw. Festphasen sind dem Fachmann bekannt und beispielsweise in EP 0344578 beschrieben.

Die Immobilisierung der markierten Nukleinsäurehybrids dauert mindestens ca. 30 Sekunden; mehrere Stunden, d.h. auch über Nacht verschlechtern das Ergebnis nicht. In der Regel sind 2 bis 10 Minuten ausreichend; bevorzugt sind ca. 5 Minuten bei einer Temperatur von ca. 37°C. Je nach Länge der Inkubationsdauer sind jedoch auch Temperaturen von 4° bis 80°C geeignet.

Von größter Wichtigkeit ist, daß nach Abschluß des Immobilisierungsvorgangs gründlich gewaschen wird, um beispielsweise nicht spezifisch gebundene markierte Nukleinsäurehybride bzw. markierte einzelsträngige RNA/DNA sowie die Nukleinsäuren spaltenden Reagenzien möglichst quantitativ zu entfernen.

Die anschließende Detektion des immobilisierten Nukleinsäurehybrids erfolgt entweder direkt über den an das Hybrid gebundenen detektierbaren Rest, der z.B. ein Chemilumineszenz- bzw. Fluoreszenzfarbstoff sein kann oder, indirekt, wenn es sich bei dem detektierbaren Rest um ein Hapten, wie z.B. Digoxigenin (DIG) handelt. Die Inkubation erfolgt in einem solchen Fall beispielsweise mit POD-markiertem (DIG)-Antikörper in ca. 30 bis 60 Minuten bei ca. 37°C und 400 rpm. Die anschließende Detektion erfolgt z.B. durch Einpipettieren einer Substratlösung wie beispielsweise Luminol/Iodphenol oder Tetramethylbenzidin und Messung des Signals Extinktion nach ca. 3 bis 30 Minuten.

Der Kit für den Nachweis bzw. die Bestimmung von einzelsträngigen spezifischen Oligo- bzw. Polynukleolidsequenzen besteht aus mindestens drei der folgenden Komponenten:

(a) Lysis- bzw. Hybridisierungspuffer,
(b) mindestens eine markierte zu der zu bestimmenden Polynukleotidsequenz komplementäre Oligo- oder Polynukleotidprobe,
(c) ein einzelsträngige Polynukleotidsequenzen in Mond bzw. Oligonukleotide spaltendes Agenz sowie einen geeigneten Verdünnungspuffer,
(d) einen Enzym-markierten Antikörper, der gegen die Markierungsgruppe der zu der zu bestimmenden Polynukleotidsequenz komplementären Oligo- bzw. Polynukleotidprobe gerichtet ist, sowie ein für das Enzym geeignetes Substrat und
(e) ein Reaktionsgefäß, welches auf der Innenseite mit einer Substanz beschichtet ist, welche mit einer zweiten Markierungsgruppe der zu der zu bestimmenden Polynukleotidsequenz komplementären Oligo- bzw. Polynukleotidprobe bindbar ist.

Als besonders geeignet hat sich ein Kit erwiesen, der drei der fünf genannten Komponenten, beispielsweise die Komponenten a, b und d oder die Komponenten a, d und e enthält.

Als besonders geeignet hat sich auch ein Kit erwiesen der vier der fünf genannten Komponenten, beispielsweise die Komponenten a, b, d, und e oder die Komponenten a, c, d, und e enthält.

Als besonders geeignet hat sich vor allem ein Kit erwiesen, der alle fünf genannten Komponenten enthält.

Als besonders geeignet hat sich darüber hinaus ein Kit erwiesen, der als einzelsträngige Polynukleotidsequenzen spaltendes Agenz eine Mischung von unterschiedlichen Spaltagenzien, wie z.B. RNase T1 und RNase A enthält. Ganz besonders vorteilhaft hat sich erfindungsgemäß erwiesen, wenn eines der in der Mischung enthaltenen Spaltagenzien mengenmäßig einen Bruchteil des/der anderen Spaltagenzien ausmacht. Der Bruchteil kann 1/5 bis maximal 1/100000 der Gesamtmenge an Spaltagenzien bedeuten.

Das erfindungsgemäße Verfahren bietet insbesondere den Vorteil der höheren Sensitivität, da überraschenderweise störende Hintergrundsignale reduziert werden konnten, der Einfachheit der experimentellen Durchführung (keine RNA-Isolierung zwingend erforderlich, keine Blotting-Schritte erforderlich) und der Automatisierbarkeit.

Zudem ist bei dem erfindungsgemäßen Verfahren von Vorteil, daß die Hybridisierungsreaktion nicht an der Festphase, sondern in Lösung erfolgt. Hybridisierungen in Lösung erfolgen effzienter und erheblich schneller.

Erläuterungen zu den Abbildungen:

Abbildung 1:

Zeigt das Ergebnis des Beispiels 1, wobei □ = + CAT , ein Gen, welches nicht endogen in eukariotischen Zellen vorkommt bedeutet, die gefüllten Symbole bedeuten "-CAT RNA". Es wurden 6,7 ng CAT RNA pro Reaktionsmulde aufgetragen und konnten bei einem Signal/Rauschen-Verhältnis von >15 nachgewiesen werden.

Die linken Signale sind auf den Verdau mit RNase T1 zurückzuführen, die rechten auf eine Mischung von RNase T1/RNase A. Im ersten Fall sind unspezifische Hybride auch durch Steigerung der RNase T1-Konzentration nicht vollständig zu beseitigen; dagegen bewirkt die Mischung bestehend aus RNase T1 und RNase A, selbst bei geringen Konzentrationen von RNase A, eine weitere deutliche Verbesserung.

Abbildung 2:

Zeigt das Ergebnis des Beispiels 2, wobei ○ = Actin und □ = CAT bedeuten.

Abbildung 3:

Fließdiagramm des erfindungsgemäßen Verfahrens.

Die folgenden Beispiele erläutern die Erfindung:

Einführung

Das erfindungsgemäße Verfahren dient zum Nachweis und zur Quantifizierung spezifischer RNA bzw. zur Analyse von Gen- und mRNA-Strukturen.

Die Technik basiert auf der Hybridisierung von z.B. Bio/DIG gelabelter antisense-RNA-Probe mit dem zu testenden RNA Pool (Probenmaterial). Komplementäre Teile der Test RNA und der Probe bilden Hybride. Mittels RNasen werden nicht hybridisierte Target- und Proben-RNAs verdaut, die Hybride bleiben intakt. Über den Biotinlabel werde die Hybride in der Streptavidin beschichteten Mikrotiterplatte gebunden und hier über ⟨DIG⟩POD nachgewiesen.

Die Bio/DIG gelabelte antisense-RNA-Probe wird durch in vitro-Transkription von Chloramphenicolacetyltransferase (CAT)-PCR-Fragmenten gewonnen, die über entsprechende Primer mit dem T7 Polymerase Promoter versehen wurden.

In den folgenden Beispielen wird die Messung von CAT-spezifischer RNA in einer Matrix aus Hefe-Gesamt-RNA mittel Ribonuclease-Protection-ELISA (RPE) beschrieben.

Beispiel 1

Das Transkriptionsvektrokonstrukt zur Herstellung von CAT-antinsense-RNA-Probe und die spezifische CAT-RNA zum In-Kontaktbringen mit den Proben stammen von der Firma Ambion. Weitere Reagenzien zur Erzeugung der Probes stammen von Boehringer Mannheim. Hefe-Gesamt-RNA (c=5 mg/ml), Hybridisierungspuffer (40 mM Pipes, 400 mM NaCl, 1 mM EDTA, 80% deionisiertes Formamide, pH 6,4), RNaseverdaupuffer (10 mM Tris-HCl, 5 mM EDTA, 300 mM NaOAc, pH 7,5), RNase A (3,5 mg/ml), RNase T1 (10 U/$\mu$l) wurden aus einem Ribonuclease Protection Kit (Gelformat, Boehringer Mannheim) verwendet.

Alle weiteren Reagenzien wie die Streptavidin beschichtete Mikrotiterplatte (MTP), ⟨DIG⟩POD-Antikörper, RNase-freier Konjugatverdünnungspuffer (40 mM $KPO_4$, 1 mM EDTA, 0,25% RSA, pH 6,8) Luminol/Iodphenolchemilumineszenzsubstrat und weiteres stammen von Boehringer Mannheim. Chemilumineszenzmessungen wurden mit dem Microplate Luminometer LP 96P von Berthold durchgeführt.

Mittels DIG RNA Labeling Kit (Boehringer Mannheim) und durch entsprechendes Ersetzen von UTP durch Bio-UTP (molares Verhältnis UTP/DIG-UTP=3/1 und UTP/Bio-UTP=20/1) werden mit je 1 $\mu$g Template DNA nach den Angaben des Herstellers ca. 20 $\mu$g CAT-antisense-RNA gewonnen. Nach Ethanolfällung und resuspendieren in je 60 $\mu$l TE Lagerung bei -70°C.

In sechs sterile Eppendorfgefäße wurden (auf Eis) je 30 $\mu$l Hybridisierungspuffer und je 2 $\mu$l Hefe-Gesamt-RNA Lösung (10 $\mu$g) pipettiert. Die Ansätze 1-3 wurden mit 0,5 $\mu$l CAT-RNA (50 ng/$\mu$l) versetzt. Bio/DIG gelabelte CAT-RNA Probe wurde mit TE auf ca. 30 ng/$\mu$l verdünnt und zu allen Ansätzen je 0,5 $\mu$l (15 ng) Probe pipettiert. Nach Denaturieren 5 min bei 90°C) wurde 4,5 Stunden bei 45°C hybridisiert und anschließend auf 20°C abgekühlt. Zu jedem Ansatz wurden 350 $\mu$l RNaseverdaupuffer pipettiert und anschließend mit RNasen wie folgt versetzt: Ansatz 1 und 4 mit je 3,5 $\mu$l RNase T1, Ansatz 2 und 5 mit je 38,5 $\mu$l RNase T1, Ansatz 3 und 6 mit je 3,5 $\mu$l RNase T1 und je 1 $\mu$l RNase A (1:20 vorverdünnt in RNaseverdaupuffer). Der RNaseverdau erfolgt bei ca. 30 Minuten bei 30°C, anschließend wird auf Eis gesetzt. Je 100 $\mu$l Ansatz wurden in eine auf 37°C vorgeheizte SA-MTP pipettiert. 5 Minuten Bindung bei 400 rpm. Anschließend wurde dekantiert und 8mal mit 0,1 x SSC gewaschen. Einpipettieren von je 100 $\mu$l ⟨DIG⟩POD Konjugat (25 mU/ml) und 30 Minuten Inkubation bei 400 rpm und 37°C. Anschließend wurde dekantiert und 3mal mit 0,1 x SSC gewaschen. Einpipettieren von je 150 $\mu$l Luminol/Iodphenol und Messung ach 3 Minuten (Integrationszeit 1 Sekunde).

| Ergebnisse | |
|---|---|
| Ansatz No. | Relative Lichteinheiten |
| 1 | 1228767 |
| 2 | 1195821 |
| 3 | 1023721 |
| 4 | 343837 |
| 5 | 260948 |
| 6 | 66162 |

Beispiel 2

Synthetische polyadenylierte "full length" CAT-sense RNA wurde wie folgt hergestellt:

- Synthese des Templates für die in vitro Transkription:
  Die primer CAT up 5'GGA TCC TAA TAC GAC TCA CTA TAG GGA GGC GAG ATT TTC AG 3' (SEQ ID NO. 1) und CAT down 5' TTT TTT TTT TTT TTT TTT TTC AGG CGT AGC 3' (SEQ ID NO. 2) wurden mit Plasmid pCATCON (Promega) als Template in einer Standard-PCR (Annealing temperature: 50,0 °C, Reagenzien von Boehringer Mannheim) zur Synthese einer DNA mit anhängigen T7 RNA Polymerase Promotor und poly A/T-tail verwendet.

- in vitro-Transkription:
100 µl Amplicon wurden mit 100%igem Ethanol gefällt, gewaschen mit 70% Ethanol und in 4 µl TE pH 8 resuspendiert. 1 µl dieser Lösung wurde in einem 40 µl in vitro Transkriptionsansatz (DIG RNA labeling kit (Boehringer Mannheim), ohne BIO/DIG-Einbau) als Template zur RNA-Synthese eingesezt. Nach Ethanolfällung wurde die Konzentration der synthetischen CAT-sense RNA mittels Gibco/DNA Mass Ladder auf ca. 8 ng/µl geschätzt.
Entsprechend wurden BIO/DIG-gelabelte antisense-RNA Probes für Actin (800 b) und CAT (241 b) hergestellt und quantifiziert (s. auch Beispiel 1).

- Ribonuclease-Protection ELISA:
Wiederfindung von CAT-sense RNA in einer Zelllysat-Matrix. $10^7$ Zellen der Zellinie K562 wurden in 1 ml Hybridisierungspuffer lysiert und die DNA durch mehrmaliges Durchziehen durch eine feine Kanüle geschert. 2 mal 5 Aliquots wurden in sterile Eppendorfcups pipettiert.
Von obiger CAT-sense RNA wurde eine log10 Verdünnungsreihe in Hybridisierungspuffer angefertigt (1:10/1:100/1:1000/1:10000). Je 2 cups Zelllysat wurden mit je 1 µl CAT-sense RNA der verschiedenen Konzentrationen (beginnend mit 800 pg/µl) und die beiden letzten cups mit je 1 µl Hybridisierungspuffer in Kontakt gebracht.
Ca. 150 pg BIO/DIG-gelabelte antisenac-RNA Probes wurden zu den in-Kontakt-gebrachten Lysaten pipettiert, 4h bei 55°C und unter Schütteln hybridisiert und mit RNaseverdau-Puffer auf das 11fache Volumen aufgefüllt. Anschließend erfolgte der Verdau mit 1,28 U/ml RNase A und 514 U/ml RNase T1 (Endkonxentration) für 40 min bei 40°C. Je 100 µl wurden in die auf 50°C vorgeheizte SA-MTP überpipettiert und 5 min bei 500 rpm gebunden. Anschließend wurde dekantiert und 8 mal mit 0,1 x SSC gewaschen. Die Detektion erfolgte mit anti-DIG POD wie in Beispiel 1 und BM Blue (Boehringer Mannheim) als Substrat (100 µl pro well, mit 25 µl 1 M Schwefelsäure nach 6 min abgestoppt, Messung bei 450 nm).

Ergebnis:

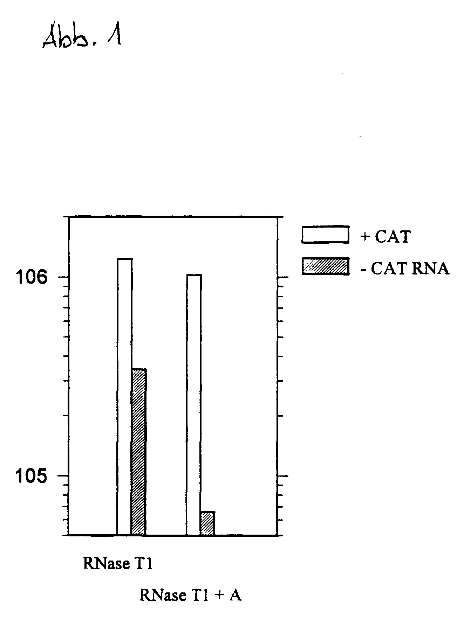

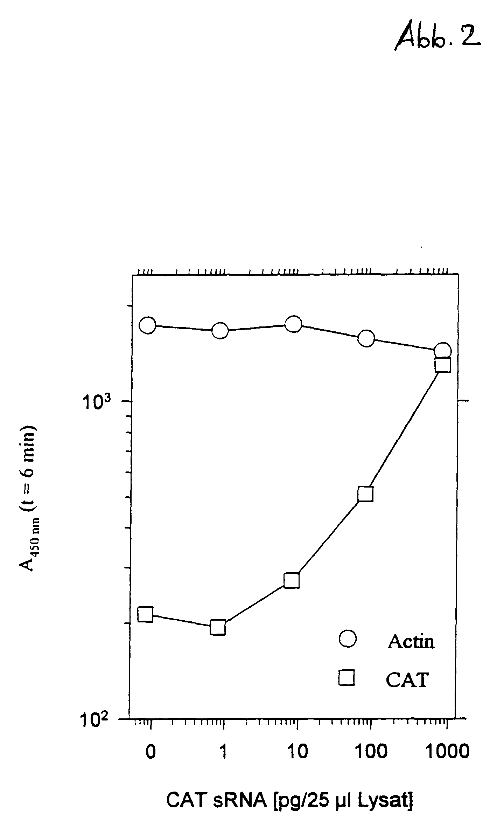

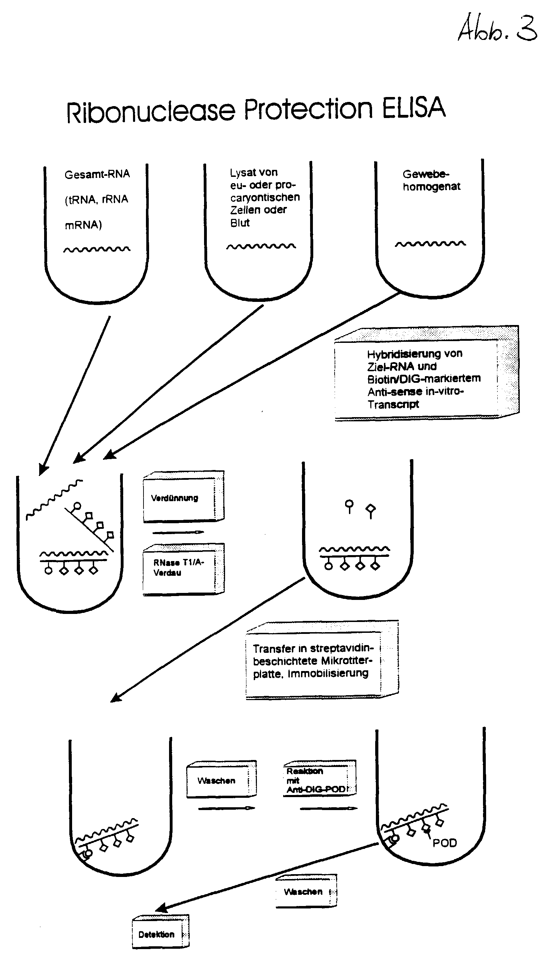

- Unterschiedliche Mengen CAT-sense RNA wurden in einer Matrix aus Zelllysat bis zu einer Konzentration von weniger als 10 ng/µl nachgewiesen (Abbildung 2).

- Die nachgewiesenen Actin-Transkriptkonzentrationen blieben erwartungsgemäß von Aliquot zu Aliquot konstant.

- Beim In-Kontakat-bringen mit einer Menge CAT-sense RNA, die der in etwa erwarteten Menge Actin-sense RNA mit Lysat entspricht, sind die erhaltenen Signale für beide Transkripte gleich hoch.

**Patentansprüche**

1. Verfahren zum Nachweis bzw. zur quantitativen Bestimmung von spezifischen Oligo- bzw. Polynukleotidsequenzen in einer Probe, welches folgende Stufen umfaßt:

a) Vereinigen einer RNA oder einzelsträngige DNA enthaltenen Probenmischung mit einem Lysis- bzw. Hybridisierungspuffer,
b) In Kontaktbringen der homogenisierten Probenlösung mit einer oder mehrerer zu der zu bestimmenden Polynukleotidsequenz im wesentlichen komplementären Oligo- oder Polynukleotid-Prob(s) bzw. entsprechende Derivate, wobei mindestens zwei unterschiedliche Markierungsreste, von denen mindestens einer eine spezifisch bindbare chemische Gruppe und einer eine detektierbare chemische Gruppe darstellt, an dieselbe Probe und an jeweils eine von verschiedenen spezifischen Probes gebunden sind,
c) Hybridisieren unter stringenten Bedingungen bei einer Temperatur von ungefähr 30° bis 80°C und innerhalb von ca. 10 Minuten bis 24 Stunden,
d) Verdünnen der erhaltenen Mischung und Zusatz eines einzelsträngige Polynukleotidsequenzen in Mono- bzw. Oligonukleotide spaltenden Agenzes,
e) Transfer der Mischung in ein Reaktionsgefäß bzw. -mulde, dessen Innenseite mit einer zumindest eine der Markierungsreste bindenden Substanz beschichtet ist,
f) Immobilisierung des mit zwei unterschiedlichen Markierungsgruppen bzw. einer Markierungsgruppe modifizierten Oligo- bzw. Polynukleotidhybrids an die Festphase und anschließendes Waschen, und
g) Detektion der immobilisierten, markierten Oligo- bzw. Polynukleotidprobe oder der nicht-immobilisierten markierten Nukleotidprobe, wobei die Detektion direkt oder über einen geeigneten Enzym-markierten Antikörper erfolgt.

2. Verfahren nach Anspruch 1, dadurch gekennzeichnet, daß der Lysis- bzw. Hybridisierungspuffer Guanidinumthiocyanat (GTC) enthält und RNase-frei ist bzw. einen RNase-Inhibitor enthält.

3. Verfahren nach einem der Ansprüche 1 oder 2, dadurch gekennzeichnet, daß das Verhältnis von Detektions- zu Bindelabel 10 bis 1 beträgt.

4. Verfahren nach einem der Ansprüche 1 bis 3, dadurch gekennzeichnet, daß als einzelsträngige Polynukleotidsequenzen spaltendes Agenz RNase T1 und/oder RNase A und/oder RNase I zugesetzt wird und ca. 2 Minuten bis 3 Stunden in einem Temperaturbereich von ca. 10° bis 80°C inkubiert wird.

5. Verfahren nach Anspruch 4, dadurch gekennzeichnet, daß ca. 10 bis 60 Minuten bei ca. 20° bis 50°C inkubiert wird.

6. Verfahren nach einem der Ansprüche 1 bis 5, dadurch gekennzeichnet, daß die Mischung vor Zusatz des einzelsträngige Polynukleotidsequenzen spaltenden Agenzes um ca. 1:2 bis 1:100 verdünnt wird.

7. Verfahren nach einem der Ansprüche 1 bis 6, dadurch gekennzeichnet, daß es sich bei dem Bindelabel um Biotin, Imminobiotin oder Avidin, bei dem Detektionslabel um Digoxigenin oder Fluoreszein und um ein Streptavidin-beschichtetes Reaktiosgefäß handelt.

8. Verfahren nach einem der Ansprüche 1 bis 7, dadurch gekennzeichnet, daß die Immobilisierung bei einer Temperatur von ca. 4° bis 80°C und innerhalb von ca. 30 Sekunden bis 24 Stunden erfolgt.

9. Verfahren nach Anspruch 8, dadurch gekennzeichnet, daß bei einer Temperatur von ca. 20° bis 50°C und innerhalb von ca. 2 bis 10 Minuten immobilisiert wird.

10. Verfahren nach einem der Ansprüche 1 bis 9, dadurch gekennzeichnet, daß die Detektion mittels eines Enzym-markierten ⟨Dioxigenin⟩-Antikörpers und einem geeigneten Substrat erfolgt.

11. Kit für den Nachweis bzw. die Bestimmung von einzelsträngigen spezifischen Oligo- bzw. Polynukleotidsequenzen dadurch gekennzeichnet, daß mindestens drei der folgenden Komponenten enthalten sind:

    (a) Lysis- bzw. Hybridisierungspuffer,
    (b) mindestens eine markierte zu der zu bestimmenden Polynukleotidsequenz komplementäre Oligo- oder Polynukleotidprobe,
    (c) ein einzelsträngige Polynukleotidsequenzen in Mono- bzw. Oligonukleotide spaltendes Agenz,
    (d) einen Enzym-markierten Antikörper, der gegen die Markierungsgruppe der zu der zu bestimmenden Polynukleotidsequenz komplementären Oligo- bzw. Polynukleotidprobe gerichtet ist, sowie ein für das Enzym geeignetes Substrat und
    (e) ein Reaktionsgefäß, welches auf der Innenseite mit einer Substanz beschichtet ist, welche mit einer zweiten Markierungsgruppe der zu der zu bestimmenden Polynukleotidsequenz komplementären Oligo- bzw. Polynukleotidprobe bindbar ist.

12. Kit nach Anspruch 11 dadurch gekennzeichnet, daß mindestens vier Komponenten enthalten sind.

13. Kit nach Anspruch 11 dadurch gekennzeichnet, daß alle fünf Komponenten enthalten sind.

14. Kit nach Anspruch 11 bis 13, dadurch gekennzeichnet, daß als einzelsträngige Polynukleolidsequenzen spaltendes Agenz RNase T1, RNase A und/oder RNase I verwendet wird.

Abb. 1

Abb. 2

Abb. 3

# Ribonuclease Protection ELISA

SEQUENZPROTOKOLL

(1)  ALLGEMEINE ANGABEN:

(i) ANMELDER:
    (A) NAME: BOEHRINGER MANNHEIM GMBH
    (B) STRASSE: Sandhofer Str. 116
    (C) ORT: Mannheim
    (E) LAND: Deutschland
    (F) POSTLEITZAHL: D-68305
    (G) TELEFON: 0621/759-3277
    (H) TELEFAX: 0621/759-4457

(ii) BEZEICHNUNG DER ERFINDUNG: Verfahren zum quantitativen
     Nachweis von spezifischen Nukleinsäuresequenzen

(iii) ANZAHL DER SEQUENZEN: 2

(iv) COMPUTER-LESBARE FASSUNG:
    (A) DATENTRÄGER: Floppy disk
    (B) COMPUTER: IBM PC compatible
    (C) BETRIEBSSYSTEM: PC-DOS/MS-DOS
    (D) SOFTWARE: PatentIn Release #1.0, Version #1.30B (EPA)

(v) DATEN DER JETZIGEN ANMELDUNG
    ANMELDENUMMER:

(2)  ANGABEN ZU SEQ ID NO: 1

(i) SEQUENZKENNZEICHEN:
    (A) LÄNGE: 41 Basenpaare
    (B) ART: Nucleotid
    (C) STRANGFORM: Einzelstrang
    (D) TOPOLOGIE: linear

(ii) ART DES MOLEKÜLS: other nuckleic acid
    (A) BESCHREIBUNG: /desc = "Primer"

(xi) SEQUENZBESCHREIBUNG: SEQ ID NO: 1

GGATCCTAAT ACGACTCACT ATAGGGAGGC GAGATTTTCA G          41

(2) ANGABEN ZU SEQ ID NO: 2

(i) SEQUENZKENNZEICHEN:
(A) LÄNGE: 30 Basenpaare
(B) ART: Nucleotid
(C) STRANGFORM: Einzelstrang
(D) TOPOLOGIE: linear

(ii) ART DES MOLEKÜLS: other nuckleic acid
(A) BESCHREIBUNG: /desc = "Primer"

(xi) SEQUENZBESCHREIBUNG: SEQ ID NO: 2

TTTTTTTTTT TTTTTTTTTT CAGGCGTAGC          30